# EUROPEAN PATENT SPECIFICATION

(11) **EP 3 296 905 B1**
(45) Date of publication and mention of the grant of the patent: **08.11.2023**
(21) Application number: 16189089.2
(22) Date of filing: 15.09.2016
(51) Int. Cl.: G16B 40/20

(54) **METHOD FOR IDENTIFYING NEW MARKERS**
VERFAHREN ZUR IDENTIFIZIERUNG NEUER MARKER
PROCÉDÉ PERMETTANT D'IDENTIFIER DE NOUVEAUX MARQUEURS

(43) Date of publication of application: 21.03.2018
(73) Proprietor: Alacris Theranostics GmbH, 14195 Berlin (DE)
(72) Inventor: LANGE, Bodo M. H., 14195 Berlin (DE); LEHRACH, Hans, 14195 Berlin (DE); WIERLING, Christoph, 14195 Berlin (DE); SHADRIN, Alexey, 14195 Berlin (DE)
(74) Representative: Arth, Hans-Lothar

(56) References cited:
- Said Blaik: "Permutation Feature Importance | Cortana Intelligence and Machine Learning Blog", , 14 May 2015 (2015-05-14), XP055349455, Retrieved from the Internet: URL:https://blogs.technet.microsoft.com/ma chinelearning/2015/04/14/permutation-featu re-importance/ [retrieved on 2017-02-24]
- A. ALTMANN ET AL: "Permutation importance: a corrected feature importance measure", BIOINFORMATICS., vol. 26, no. 10, 12 April 2010 (2010-04-12), pages 1340-1347, XP055349645, GB ISSN: 1367-4803, DOI: 10.1093/bioinformatics/btq134
- Ryo Takata ET AL: "Validation study of the prediction system for clinical response of M-VAC neoadjuvant chemotherapy", CANCER SCIENCE, vol. 98, no. 1, 1 January 2007 (2007-01-01), pages 113-117, XP055663655, JP ISSN: 1347-9032, DOI: 10.1111/j.1349-7006.2006.00366.x

## Description

### Specification

The present invention relates to a method of identifying biomarkers of a responder and a non-responder with a mechanistic model, provided with individual parameters of the patient and experimentally measured target characteristic of the patient. A patient is assumed to be either a responder or a non-responder depending on the value of its experimentally measured target characteristic. The used mechanistic model allows prediction of the target characteristic of a patient based on predefined parameters corresponding to this patient.

### Background of the invention

Mathematical modelling of complex systems is extremely helpful and became very important in many areas, such as medical, surgical, industrial, chemical, biological, agricultural and technical areas, to predict outcome and efficiency of processes as well as properties of products. Such modeling allows optimizing complex systems in order to maximize outcome and efficiency of processes and to achieve desired properties of the products. One important aspect of any such mathematical model is to understand the effects of changes to various model parameters.

For instance, such models also make an important contribution to drug development processes as well as to predicting treatment responses, by modeling responses of biological systems and also by determining relevant (molecular) processes and parameters of the plethora of parameters involved in a complex biological system.

Those relevant parameters among the plethora of parameters involved in a complex system that influence the outcome of the model are especially useful as markers.

Takata et al. (Cancer Sci 2007, 98(1), 113-117) report on a prediction system for clinical response of M-VAC neoadjuvant chemotherapy for invasive bladder cancers on basis of permutation analysis. Genes that were expressed significantly different between responders and non-responders of M-VAC neoadjuvant chemotherapy were identified by randomly permutating the expression level of a gene between responders and non-responders and calculating a discrimination score.

"Permutation Feature Importance" (PFI) is a feature selection method, i.e. a feature ranker based on permutation importance algorithm compatible with classification and regression models (https://blogs.technet.microsoft.com/machinelearning/2015/04/14/permutation-feature-importance/). Given a trained model, a test dataset, and an evaluation metric, the PFI module creates a random permutation of a feature column (shuffles the values of that column) and evaluates the performance of the input model on the modified dataset. This is done iteratively for each of the feature columns, one at a time. The module then returns a list of the feature variables and their corresponding importance scores. The importance score is defined as the reduction in performance after shuffling the feature values.

However, limited experimental data and especially computationally expensive and inaccurate or misleading analysis methods of mathematical models are the bottlenecks in identifying relevant processes and parameters as markers indicating a response or specific condition or state of the studied system.

It is the objective of the present invention to provide a method of identifying markers of a system's condition or response, in particular markers of a specimen's state.

The objective of the present invention is solved by the teaching of the independent claims. Further advantageous features, aspects and details of the invention are evident from the dependent claims, the description, the figures, and the examples of the present application.

### Brief description of the invention

The present invention relates to a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

In one embodiment of the computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment;
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful, and wherein the deviation is a statistically significant deviation.

In one embodiment of the computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment;
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful, and wherein the responder and the non-responder are patients suffering from a proliferative disease.

In a preferred embodiment, the proliferative disease is selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, and tongue cancer.

In one embodiment of the computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment;
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful, and wherein the individual parameters of the responder and the individual parameters of the non-responder are selected from gene expression data, mutation data, gene allele and abundance of proteins.

In one embodiment of the computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment;
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful, and wherein the mechanistic model is capable of predicting the target characteristic of the responder from the individual parameters of the responder and wherein the mechanistic model is capable of predicting the target characteristic of the non-responder from the individual parameters of the non-responder.

In one embodiment of the computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment;
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful, and wherein the mechanistic model consists of a set of ordinary differential equations.

### Description of the invention

### Definitions

The term "**experimentally measured target characteristic**" as used herein refers to any feature of a specimen that can be measured accurately and reproducibly. Any functional, physiological, behavioral, chemical, physical or biochemical observable of a specimen that indicates its condition or response to environmental influences may be suitable as target characteristic.

The target characteristic can be represented by discrete or continuous one dimensional real valued variables that can take values from the interval [A', B'], where both A' and B' are real numbers and each of A' and B' can be infinite. One end of the interval represents state A (responder), another end represents state B (non-responder), regardless which end represents which state. Thus, A' may represent state A and B' may represent state B, or A' may represent state B and B' may represent state A. This assumption holds true for the following, even if it is not explicitly mentioned. For a person skilled in the art, it is obvious that the naming of specimen's states A and B is arbitrary. They may also be called state 1 and state 2 , first state and second state or responder and non-responder, etc. Furthermore, for classification whether a specimen is in state A or state B or none of them, two thresholds t1 and t2 are defined in such a way that the following condition is satisfied: A' ≤ t1 ≤ t2 ≤ B', wherein at least one inequality is strict. The values for A' and B' may be chosen arbitrarily as long as the above mentioned condition is true. In general A' and B' reflect the lowest experimentally measured target characteristic and the highest experimentally measured target characteristic that a specimen can reach.

The **specimen** is an individual patient suffering from the same disease and receiving equal treatment. The experimentally measured target characteristic is the body temperature. A' corresponds to a very low body temperature the patient can reach and is 20 °C. B' corresponds to a very high body temperature the patient can reach and is 44 °C. Both thresholds t1 and t2 are 37.5 °C. Thus, a patient with a body temperature below 37.5 C is a specimen in state A, i.e. the patient has no fever. A patient with a body temperature above or equal 37.5 C is a specimen in state B, i.e. the patient has fever. In another example, the specimen is a cancer cell line, which is treated with a drug at different concentrations. The experimentally measured target characteristic is the cancer cell growth rate of the treated specimen compared to an untreated specimen. Thus, A' corresponds to a very low growth rate compared to the untreated specimen and B' corresponds to a very high growth rate of the treated specimen. Threshold t1 is set 30% and t2 is set to 70%. Consequently, a treated cancer cell line with a growth rate below 30% (t1) compared to an untreated cancer cell line is a specimen in state A, i.e. a cancer cell line which responds to the drug treatment (responder). A treated cancer cell line with a growth rate above 70% (t2) compared to an untreated cancer cell line is a specimen in state B, i.e. a cancer cell line which does not respond to the drug treatment (non-responder). A treated cancer cell line with a growth rate above 30% and below 70% is neither a responder nor a non-responder.

Thus, a specimen is unambiguously classified as a responder (or non-responder) if its experimentally measured target characteristic is below or equal the defined threshold t1. A specimen is classified unambiguously as a non-responder (or responder) if its experimentally measured target characteristic is above or equal the defined threshold t2. Consequently no specimen can be a responder and non-responder simultaneously. However, when t1 < t2, there may be specimens having an experimentally measured target characteristic between thresholds t1 and t2 and are thus neither responder nor non-responder. Also, when t1 = t2 and A' < t1 = t2 < B', specimens having an experimentally measured target characteristic that is equal to t1 and t2 are neither responder nor non-responder. These specimens may be regarded as unclassified and should not be used within the inventive method. Any method for determining thresholds t1 and t2 as well as any method for selecting a classification strategy may be used in the inventive method.

Thus, a "**responder**" as used herein is defined as a specimen whose experimentally measured target characteristic is below or equal the defined threshold t1 (or above or equal the defined threshold t2) and a "**non**-**responder**" as used herein is defined as a specimen whose experimentally measured target characteristic is above or equal the defined threshold t2 (or below or equal the defined threshold t1).

In one embodiment of the present invention the threshold t1 equals threshold t2. In another embodiment thresholds t1 and t2 are set to A' + (B' - A')/2. In another embodiment threshold t1 is set to A' + x, wherein x is preferably at most 90%, more preferably at most 80%, more preferably at most 70%, more preferably at most 60%, more preferably at most 50%, more preferably at most 40%, even more preferably at most 30% and most preferably at most 20% of (B' - A')/2. In still another embodiment threshold t2 is set to B' - y, wherein y is at most 90%, preferably at most 80%, more preferably at most 70%, more preferably at most 60%, more preferably at most 50%, more preferably at most 40%, even more preferably at most 30% and most preferably at most 20% of (B' - A')/2. In a further embodiment the difference t2 - t1 is preferably at least 10%, more preferably at least 20%, more preferably at least 30%, more preferably at least 40% and even more preferably at least 50% of (B' - A')/2.

The term "**set of specimen**" as used herein refers to a group of specimens comprising of at least one responder and at least one non-responder.

The computer-implemented method of identifying biomarkers of the present invention uses a mechanistic mathematical model suitable for predicting the target characteristic of a specimen. To this end, the model is provided with a set of parameters, which can be obtained experimentally, from databases or upon optimization of the model. In contrast, the experimentally measured target characteristic is not a parameter of the model as it represents the ideal prediction of the mathematical model.

The parameters of the mathematical model used in the inventive method can be divided into four types: common parameters, individual parameters, predefined parameters and free parameters.

"**Common parameters**" as used herein refer to parameters of the mathematical model whose values are identical for all specimens of a set of specimens. They do not depend on the specific individual specimen and are thus not affected by variations occurring between individual specimens. For example, when the specimen is a patient having a disease and which is treated with a drug, the drug concentration as model parameter has the same value for all patients, because it is determined by the treatment and not by the individual specimen. Common parameters may be for instance molecular properties such as drug concentration, protein decay rate, dissociation constants of proteins, etc. They may be obtained experimentally or from knowledge databases (predefined common parameters) or computationally by the mathematical model (free common parameters).

On the other hand "**individual parameters**" as used herein refer to parameters of the mathematical model whose values can be different or are different for different specimens. Thus, values of individual parameters may vary from specimen to specimen. They also may be obtained experimentally or from knowledge databases (predetermined individual parameters) or computationally by the mathematical model (free individual parameters). These individual parameters of a specimen may include gene expression data or mutation data, gene allele, expression strength of a gene, abundance of proteins, symptoms such as inflammations, treatment, etc.

Parameters of the mathematical model used in the inventive method may also be divided according to the origin of their values independently from dividing them into common and individual parameters.

The term "**predefined parameters**" as used herein refers to those common parameters and individual parameters, whose values are obtained from experimental data or from knowledge databases before they enter the mathematical model. Experimentally, the predefined parameters may be collected from various samples of a specimen or the specimen itself, such as blood, plasma, serum, tissues, tumor tissue and cerebrospinal fluid, if applicable. In case the specimen is a human or an animal the predefined parameters may be obtained by bronchoalveolar lavage, biopsy, gene expression analysis and further diagnostic methods. Predefined parameters may also be obtained from databases like Ensembl, Reactome, KEGG, BioCyc or the Catalogue of Somatic Mutations in Cancer (COSMIC), etc.

In the following "predefined parameters" is used instead of explicitly mentioning predefined common parameters and predefined individual parameters for convenience reasons.

In contrast to the predefined parameters, the "**free parameters**" as used herein refer to those common parameters and individual parameters of the mathematical model whose values cannot be obtained experimentally or from databases or from literature. Instead, they are preferably determined *in silico* in such a way that the mathematical model used in the present invention shows the desired behavior, i.e. the agreement between the experimentally measured target characteristic and the predicted target characteristic is optimized.

In the following "free parameters" is used instead of explicitly mentioning free common parameters and free individual parameters for convenience reasons.

Thus, the essential characteristic of the free parameters including free common parameters and free individual parameters of the mathematical model cannot be measured, cannot be obtained experimentally, and cannot be obtained from databases or from literature and must be calculated by the model.

The term "**biomarker**" as used herein refers to the individual parameter or subset of individual parameters of the mathematical model that can be used to indicate a specimen's state. Biomarkers identified by the method according to the present invention are markers indicating whether a specimen is a responder or not and/or are markers indicating whether a specimen is a non-responder or not and/or are markers indicating whether a specimen is a responder or a non-responder.

In other words, a biomarker of a responder according to the present invention is that swapped subset of individual parameters of the responder that causes a deviation between the experimentally measured target characteristic of the responder and the predicted target characteristic for the swapped subset of individual parameters of the responder towards the experimentally measured target characteristic of the non-responder. A biomarker of a non-responder according to the present invention is that swapped subset of individual parameters of the non-responder that causes a deviation between the experimentally measured target characteristic of the non-responder and the predicted target characteristic for each swapped subset of individual parameters of the non-respondertowards the experimentally measured target characteristic of the responder.

A specimen's state may be regarded as its condition (e.g. medical, physiological, biochemical, clinical, physical, technical, etc.) or response (e.g. medical, functional, physiological, and behavioral, etc.) to environmental influences, such as chemical, physical or biological hazards, therapeutics, and treatments, etc. Consequently, markers identified by the inventive method may be used to determine the condition in which the specimen is in. In addition, biomarkers identified by the inventive method may be used to determine the condition in which the specimen will be in. Furthermore, biomarkers identified by the inventive method may be used to determine how a specimen will respond to environmental influences. Also, biomarkers identified by the inventive method are not the experimentally measured target characteristic.

In one embodiment of the present invention, the inventive method may be used to identify biomarkers that are selected from the following non-exhaustive list: biochemical markers, DNA biomarkers, molecular biomarkers, imaging biomarkers, nonimaging biomarkers, pharmacogenomic biomarkers, cancer biomarkers, pharmacokinetic biomarkers, pharmacogenetic biomarkers, pharmacodynamic biomarkers, metabolomic biomarkers, cytogenic biomarkers, autism biomarkers, RNA biomarkers, inflammatory markers, proteomic or protein biomarkers, transcriptomic biomarkers, genomic biomarkers, serum biomarkers, blood biomarkers, cerebrospinal fluid (CSF) biomarkers, histo-cytopathological biomarkers, synthetic biomarkers, cell-free biomarkers, cell specific biomarkers, combination or integrative biomarkers, behavioral markers, biomarkers for bipolar disorder, endophenotypes, alkane biomarkers, oil biomarkers, petroleum biomarkers, environmental biomarkers, direct and indirect biomarkers, ecological biomarkers, surrogate markers, disease markers, disease risk biomarkers, diagnostic markers, prognostic markers, antecedent biomarkers, screening biomarkers, staging biomarkers, predictive markers, efficacy-response biomarkers, efficacy biomarkers, exclusion biomarkers, exploratory biomarkers, exposure biomarkers, clinical biomarkers, markers of target engagement, stratification biomarkers, Type 0 biomarkers, Type I biomarkers, Type II biomarkers, cardiac markers, anemia biomarkers, autoimmune disease biomarkers, bone disease biomarkers, diabetes biomarkers, endocrinology biomarkers, gastroenterology biomarkers, infectious diseases biomarkers, inflammation markers, immunity markers, proliferative biomarkers, lipid metabolism markers, nephrology biomarkers, nephropathy biomarkers, oncology biomarkers, tumor markers, thyroid markers, neurological biomarkers, biomarkers of neurodegenerative disorders, central nervous system (CNS) biomarkers, gastrointestinal disease biomarkers, hematology biomarkers, physiological biomarkers, aging biomarkers and wellness biomarkers.

The present invention is further related to a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively replacing a subset of individual parameters between the responder and the non-responder and predicting target characteristics for each replaced subset of individual parameters of the responder and predicting target characteristics for each replaced subset of individual parameters of the non-responder;
II) Determining deviations between the experimentally measured target characteristic of the responder and the predicted target characteristics for each replaced subset of individual parameters of the responder and determining deviations between the experimentally measured target characteristic of the non-responder and the predicted target characteristics for each replaced subset of individual parameters of the non-responder;

wherein the replaced subset of individual parameters of the responder that causes a deviation of the predicted target characteristic in direction of the experimentally measured target characteristic of the non-responder is a biomarker of a responder; wherein the replaced subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

The present invention is also related to computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively substituting a subset of individual parameters between the responder and the non-responder and predicting target characteristics for each substituted subset of individual parameters of the responder and predicting target characteristics for each substituted subset of individual parameters of the non-responder;
II) Determining deviations between the experimentally measured target characteristic of the responder and the predicted target characteristics for each substituted subset of individual parameters of the responder and determining deviations between the experimentally measured target characteristic of the non-responder and the predicted target characteristics for each substituted subset of individual parameters of the non-responder;

wherein the substituted subset of individual parameters of the responder that causes a deviation of the predicted target characteristic in direction of the experimentally measured target characteristic of the non-responder is a biomarker of a responder;
wherein the substituted subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

The present invention also relates to a method of computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping one individual parameter at a time between the responder and the non-responder and predicting target characteristics for each swapped individual parameter of the responder and predicting target characteristics for each swapped individual parameter of the non-responder;
II) Determining deviations between the experimentally measured target characteristic of the responder and the predicted target characteristics for each swapped individual parameter of the responder and determining deviations between the experimentally measured target characteristic of the non-responder and the predicted target characteristics for each swapped individual parameter of the non-responder;

wherein the swapped individual parameter of the responder that causes a deviation of the predicted target characteristic in direction of the experimentally measured target characteristic of the non-responder is a biomarker of a responder;
wherein the swapped individual parameter of the non-responder that causes a deviation of the predicted target characteristic in direction of the experimentally measured target characteristic of the responder is a biomarker of a non-responder; wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

Step I) could alternatively read as follows:
I) Iteratively swapping one individual parameter between the responder and the non-responder and predicting target characteristics for each swapped individual parameter of the responder and predicting target characteristics for each swapped individual parameter of the non-responder.

That deviation between the experimentally measured target characteristic and the predicted target characteristic is preferably a statistically significant deviation.

The mathematical **mechanistic models** used in the present invention are preferably *in silico* models representing a specimen and are further capable to predict whether a specimen is a responder or a non-responder based on its individual parameters. Thus, by entering the predefined parameters of a responder or a non-responder into the mechanistic model, the predicted target characteristics of the responder or non-responder are obtained, which are after optimization of free parameters of the model nearly identical to the experimentally measured target characteristic of the responder or non-responder. While the mechanistic model used in the present invention is specific for the regarded patient, the mechanistic model only differs for a responder or a non-responder in the entered individual parameters and the predicted target characteristic of the responder or the non-responder.

Moreover, the present invention relates to a computer-implemented method of identifying a biomarker of a responder and a biomarker of a non-responder with a mechanistic *in silico* model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

Also disclosed herein is a method to identify new markers by swapping individual parameters between the responder and the non-responder and predicting target characteristics for the swapped subset of individual parameters of the responder and the non-responder by a mechanistic model.

The herein used term "**swapping**" refers to the interchange or permutation of a subset of individual parameters between the mathematical model of the responder and the mechanistic model of the non-responder.

A "**subset** of individual parameters" as used herein refers to 1, 2, 3, 4, 5, several, some, a fraction, but not to the majority of all the individual parameters of a specimen and refers definitely not to all individual parameters. A subset of individual parameters consists preferably of no more than 50%, more preferably of no more than 40%, even more preferably of no more than 30% and most preferably of no more than 20% of all individual parameters.

Subsets of individual parameters as used in the present invention may be disjoint or not, i.e. each individual parameter may be only part of one subset or part of several subsets. Swapping means that a subset of individual parameters of the responder is set to a subset of individual parameters of the non-responder and that the same subset of individual parameters of the non-responder is set to a subset of individual parameters of the responder. Thus, after a swap the mechanistic model of the responder has a subset of individual parameters of the non-responder and the mechanistic model of the non-responder has a subset of individual parameters of the responder. In other words, after a swap the mechanistic model of the former responder contains one or more individual parameter(s) of the non-responder and the mechanistic model of the former non-responder contains one or more individual parameter(s) of the responder.

Swapping does not mean that all or the majority of individual parameters are permuted at once between the responder and the non-responder. Instead, the individual parameters or subsets of individual parameters are swapped iteratively. **Iteratively swapping** means that all subsets of individual parameters to be swapped are swapped successively and not simultaneously between the responder and the non-responder, thus resulting for each swap in a different mechanistic model of the responder and the non-responder.

Thus, a further aspect of the present invention is directed to a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Successively swapping a single subset of individual parameters of all subsets of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

For each swapped subset of individual parameters of the responder and the non-responder the predicted target characteristic of the responder and the non-responder are determined.

Also disclosed herein is a method to determine **deviations** between the experimentally measured target characteristic of the responder and the non-responder and the predicted target characteristics for each swapped subset of individual parameters of the responder and the non-responder in order to identify markers. The iteratively swapping of subsets of individual parameters has modified the mechanistic model of the responder and the non-responder in such a way, that the responder becomes more of a non-responder and the non-responder becomes more of a responder. Consequently, by swapping all individual parameters of the responderand the non-responder simultaneously, the mechanistic model of the responder becomes the mathematical model of the non-responder before the swapping and vice versa. In addition, the predicted target characteristic for all simultaneously swapped individual parameters of the responder would be nearly identical to the experimentally measured target characteristic of the non-responder and the predicted target characteristic for all simultaneously swapped individual parameters of the non-responder would be nearly identical to the experimentally measured target characteristic of the responder. However, this does not mean that each predicted target characteristic lies between the experimentally measured target characteristic of the responder and the experimentally measured target characteristic of the non-responder.

These deviations are determined by calculating the differences between the experimentally measured target characteristic of the responder and the predicted target characteristics for each swapped subset of individual parameters of the responder that lie between the experimentally measured target characteristic of the responder and the experimentally measured target characteristic of the non-responder as well as by calculating the differences between the experimentally measured target characteristic of the non-responder and the predicted target characteristics for each swapped subset of individual parameters of the non-responder that lie between the experimentally measured target characteristic of the responder and the experimentally measured target characteristic of the non-responder .

The deviations between the experimentally measured target characteristic of the responder and the predicted target characteristics for each swapped subset of individual parameters of the responder as well as between the experimentally measured target characteristic of the non-responder and the predicted target characteristics for each swapped subset of individual parameters of the non-responder that lie between the experimentally measured target characteristic of the responder and the experimentally measured target characteristic of the non-responder, indicate, which subsets of individual parameters have an influence on the specimen's state and can thus be identified according to the present invention as biomarkers of a responder or non-responder.

That deviation between the experimentally measured target characteristic and the predicted target characteristic is preferably a statistically significant deviation.

The deviation is statistically significant when a ***p*-value** (*p*-value is a function of the observed sample results relative to a statistical model which measures how extreme the observation is) is less than the **significance level α**. Normally the significance level α is chosen to be a value between 0.01 (1%) and 0.05 (5%). The *p*-value is the probability of obtaining at least as extreme results given that the null hypothesis is true whereas the significance level α is the probability of rejecting the null hypothesis given that it is true. Equivalently, when the null hypothesis specifies the value of a parameter, the data are said to be statistically significant at given confidence level γ = 1 - α, when the computed confidence interval for that parameter *fails* to contain the value specified by the null hypothesis.

In an alternative embodiment the deviation is significant when it preferably belongs to 10%, more preferably to 5%, even more preferably to 3% and most preferably to 1% of the largest deviations between the experimentally measured target characteristic of the responder and non-responder and the predicted target characteristics for each swapped subset of individual parameters of the responder and non-responder that lie between the experimentally measured target characteristic of the responder and the experimentally measured target characteristic of the non-responder. In other words, a swapped subset of individual parameters of the responder causing a deviation which belongs preferably to 10%, more preferably to 5%, even more preferably to 3% and most preferably to 1% of the largest deviations between the experimentally measured target characteristic of the responder and the predicted target characteristics for the swapped subset of individual parameters of the responder that lie between the experimentally measured target characteristic of the responder and the experimentally measured target characteristic of the non-responder, is regarded as a biomarker of a responder. Consequently, a swapped subset of individual parameters of the non-responder causing a deviation which preferably belongs to 10%, more preferably to 5%, even more preferably to 3% and most preferably to 1% of the largest deviations between the experimentally measured target characteristic of the non-responder and the predicted target characteristics for the swapped subset of individual parameters of the non-responder that lie between the experimentally measured target characteristic of the responder and the experimentally measured target characteristic of the non-responder, is regarded as a biomarker of a non-responder.

In another embodiment of the invention, new biomarkers identified by the inventive method are subsets of individual parameters of the responder and the non-responder that cause upon iteratively swapping a difference between the experimentally measured target characteristic of the responder and the predicted target characteristic of the swapped subset of individual parameters of the responder and between the experimentally measured target characteristic of the non-responder and the predicted target characteristic of the swapped subset of individual parameters of the non-responder of at least 10%, more preferable at least 15% and most preferable of at least 20% of the difference between the experimentally measured target characteristic of the responder and the experimentally measured target characteristic of the non-responder.

Thus, the present invention is further directed to a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder of at least 10%, more preferable at least 15% and most preferable of at least 20% of the difference between the experimentally measured target characteristic of the responder and the experimentally measured target characteristic of the non-responder is a biomarker of a responder;
wherein the swapped subset of individual parameters of the non-responder that causes a deviation of the predicted target characteristic in direction of the experimentally measured target characteristic of the responder of at least 10%, more preferable at least 15% and most preferable of at least 20% of the difference between the experimentally measured target characteristic of the responder and the experimentally measured target characteristic of the non-responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

Also, the present invention is further directed to a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder that preferably belongs to 10%, more preferably to 5%, even more preferably to 3% and most preferably to 1% of the largest deviations between the experimentally measured target characteristic of the responder and the predicted target characteristics for each swapped subset of individual parameters of the responder is a biomarker of a responder;
wherein the swapped subset of individual parameters of the non-responder that causes a deviation of the predicted target characteristic in direction of the experimentally measured target characteristic of the responder that preferably belongs to 10%, more preferably to 5%, even more preferably to 3% and most preferably to 1% of the largest differences between the experimentally measured target characteristic of the non-responder and the predicted target characteristics for each swapped subset of individual parameters of the non-responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

The method according to the present invention of identifying biomarkers of a responder and non-responder with a mechanistic model has several advantages over the prior art methods like local and global sensitivity analysis methods. While the local sensitivity analysis approaches only allow studying the local behavior of the mathematical model, i.e. the influence of small changes in individual parameters on predicted target characteristic, the inventive method can be used to explore the behavior of the predicted target characteristic upon non-local changes in the individual parameters. On the other hand global sensitivity analysis approaches such as variance-based methods are very time-consuming and in general not feasible for large models including several hundreds of parameters.

Due to the iteratively swapping applied in the inventive method an algorithmically simple approach is disclosed herein which avoids time-consuming calculations und is thus feasible even for huge mathematical models.

In another embodiment of the present invention the method of computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder that preferably belongs to 10%, more preferably to 5%, even more preferably to 3% and most preferably to 1% of the largest deviations between the experimentally measured target characteristic of the responder and the predicted target characteristics for each swapped subset of individual parameters of the responder is a biomarker of a responder;
wherein the swapped subset of individual parameters of the non-responder that causes a deviation of the predicted target characteristic in direction of the experimentally measured target characteristic of the responder that preferably belongs to 10%, more preferably to 5%, even more preferably to 3% and most preferably to 1% of the largest differences between the experimentally measured target characteristic of the non-responder and the predicted target characteristics for each swapped subset of individual parameters of the non-responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.
wherein the responder is the patient with the lowest experimentally measured target characteristic and the non-responder is the patient with the highest experimentally measured target characteristic of a set of patients; or
wherein the responder is the patient with the highest experimentally measured target characteristic and the non-responder is the patient with the lowest experimentally measured target characteristic of a set of patients.

In step I of the inventive method the iteratively swapping of subset of individual parameters and the predicting the target characteristics for each swapped subset of individual parameters can be performed in parallel or successively. The target characteristic can be predicted after each swapping step, or at first all subsets of individual parameters are swapped iteratively and subsequently the target characteristics for all swapped subsets of individual parameters can be predicted. Thus, step I of the inventive method is not restricted to the order in which the iteratively swapping and predicting target characteristics are performed.

However, predicting target characteristics is in general the more time-consuming and computationally more intensive step compared to the iteratively swapping and it is therefore preferred to first iteratively swap all subsets of individual parameters and subsequently predict the target characteristics for all swapped subsets of individual parameters in parallel in order to speed up the inventive method.

Thus, in one embodiment of the present invention step I) of the inventive method includes the following two steps:
a) Iteratively swapping a subset of individual parameters between the responder and the non-responder; and
b) Subsequently predicting target characteristics for each swapped subset of individual parameters of the responder and/or for each swapped subset of individual parameters of the non-responder.

In one embodiment of the present invention only the target characteristics for each swapped subset of individual parameters of the responder are predicted in step I) and in step II) of the inventive method the deviations between the experimentally measured target characteristic of the responder and the predicted target characteristic for each swapped subset of individual parameters of the responder are predicted, wherein the swapped subset of individual parameters of the responder that causes a significant deviation between the experimentally measured target characteristic of the responder and the predicted target characteristic of the responder is a marker of a responder.

In another embodiment of the present invention only the target characteristics for each swapped subset of individual parameters of the non-responder are predicted in step I) and in step II) of the inventive method the deviations between the experimentally measured target characteristic of the non-responder and the predicted target characteristic for each swapped subset of individual parameters of the non-responder are predicted, wherein the swapped subset of individual parameters of the non-responder that causes a significant deviation between the experimentally measured target characteristic of the non-responder and the predicted target characteristic of the non-responder is a marker of a non-responder.

It is envisioned that the inventive method may be repeated for a different pair of responder and non-responder of the same set of specimen in order to enhance the statistical significance of the identified markers.

The mathematical mechanistic model used in the method of identifying a biomarker according to the present invention is preferably an *in silico* model representing a specimen, i.e. calculating predictions with a mathematical model used in the inventive method requires to perform more operations than are possible to conduct by human in reasonable time and thus predictions with the mathematical model can only be performed with a computer. Consequently, the method according to the invention is a computer-aided method of identifying a biomarker.

The inventive method is not restricted to a specific mathematical mechanistic model. In principle, any mechanistic model may be applied in the inventive method that is able to predict the target characteristic of a specimen based on its individual parameters. For instance, when a specimen is a patient in anti-tumor treatment a selected mathematical model may be able to predict changes in key components, like expression of specific genes, alteration in abundance of specific growth factors in autocrine loops and inactivation of tumor suppressors, under different conditions like stimulation with growth factors, mutations, different drugs and drug combinations at different concentration. A selected model may include signaling networks, transcriptional control networks, metabolic networks, sensory and homeostatic networks, degradational networks, regulatory networks, inter-cellular interaction networks mediated by e.g. receptor-ligand action, permeable contacts like tight junctions, host-pathogen interactions as well as any other interactions between cells or organisms, examples of which are cellular growth and differentiation networks, angiogenic networks, wound healing networks, inflammatory and immune response networks as well as the complex networks of inter-cellular or inter-organismal interaction that result in functioning tissues, organs, organisms and organismal communities.

A mechanistic model suitable for the present invention may be for instance obtained from the BioModels Database or from the CellML Model Repository or may be developed with the PyBioS system.

A molecular kinetics model describes the time-dependent development of a biological system in terms of kinetic laws, kinetic constants and starting concentrations. The kinetic processes are represented as ordinary differential equations (ODE).

In the context of the invention the mechanistic model is used to predict a specimen's target characteristic from its individual parameters. The used mathematical mechanistic model can be a static or dynamical model.

However, the predefined parameters in most cases do not cover all parameters of the mathematical mechanistic model. These unknown parameters which are not covered by experimental data are called "free parameters". Depending on the concrete used mathematical model the free parameters may reflect kinetic parameters or parameters of activation functions. Thus, the free parameters of the herein used mathematical mechanistic model must be determined first in order to predict the target characteristic from the individual parameters of a specimen.

The free parameters of the mathematical mechanistic model may be determined by optimization techniques known to a skilled person from the predefined parameters and the experimentally measured target characteristic. The mathematical mechanistic model is optimized in such a way, that the free parameters are varied in order to minimize the difference between the predicted target characteristic and the experimentally measured target characteristic. Therefore, the free parameters are optimized in the sense that the obtained mathematical model is the model which reproduces best the target characteristic, i.e. the difference between the experimentally measured target characteristic and the predicted target characteristic has been minimized. This minimization may involve continuous optimization, i.e., minimization of said difference, during predicting the target characteristic. The term "optimization" relates to optimization of the above defined free parameters, such as kinetic constants and/or starting concentrations, by using for instance non-linear regression type approaches. In one embodiment the free parameters are determined by a Monte-Carlo simulation.

Preferably but not exclusively, the agreement between predicted and experimentally measured target characteristic is optimized for all available patients, i.e. responder and non-responder. Thus, in one embodiment of the present invention the optimized set of free parameters is identical for all patients, i.e. responder and non-responder. In another embodiment the optimized set of free parameters differs from patient to patient.

### Biomarker of a patient's responsiveness to the treatment

A further aspect of the present invention is related to a method of identifying a biomarker of a patient's responsiveness to the treatment of the disease the patient is suffering from. Therefore, specimens are patients suffering from the same disease and receiving equal treatment. From all patients relevant medical and biological data, including concentrations, absence and/or presence of lesions, mutations, gene alternations, symptoms such as inflammations, treatment data, gene expression data, drug concentration and irradiation in form of predefined parameters and experimentally measured target characteristics (as defined above) are available from before and during the treatment.

Accordingly, based on their experimentally measured target characteristic each patient's state is determined (as described above). In this case the experimentally measured target characteristic reflects the response to the specific treatment, i.e. indicates how effective the treatment for the single patient is. For instance, in anti-tumor therapy the response data may be the ratio between the size of the treated tumor and the size of the untreated control tumor, the so-called T/C value. T/C values may be derived from the patient himself or from patient-derived xenograft (PDX) mouse models, i.e. in the latter case cells from the tumor are implanted into a mouse of a specific mouse strain and the tumor is growing once without (untreated control) and once with drug treatment (treated tumor). The patients are classified as responder, non-responder or none of them. Patients that are neither classified as responder nor as non-responder are ignored. The classification as responder or non-responder may also be performed as follows: Responder and non-responder are two patients from a set of patients having the same disease and receiving equal treatment, that have a difference in experimentally measured target characteristic of preferably at least 70%, more preferably of at least 80% and especially preferably of at least 90% of the difference between the weakest responding patient and the strongest responding patient of the set of patients, wherein the responder is the patient that shows a stronger response to the treatment compared to the other patient and the non-responder is the patient that shows a weaker response to the treatment compared to the other patient.

In other words, a responder is the stronger responding patient of a pair of patients, which has a difference in experimentally measured target characteristic that is preferably at least 70%, even more preferably at least 80% and especially preferably at least 90% of the difference between the weakest responding patient and the strongest responding patient; and a non-responder is the worse responding patient of a pair of patients, which has a difference in experimentally measured target characteristic that is preferably at least 70%, even more preferably at least 80% and especially preferably at least 90% of the difference between the weakest responding patient and the strongest responding patient of a set of patients. For instance, in anti-tumor treatment a responder is the patient whose T/C value obtained from PDX is lower (e.g. 0.1) compared to a patient with a high T/C value obtained from PDX (e.g. 0.95) which is a non-responder.

The responder is preferably a single patient or a group of patients which exhibits in comparison to the non-responder a difference in the experimentally measured target characteristic which indicate that the treatment of the disease of the responder is successful.

The non-responder is preferably a single patient or a group of patients which exhibits in comparison to the responder a difference in the experimentally measured target characteristic which indicate that the treatment of the disease of the non-responder is not successful.

Thus, a computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder according to the present invention comprises the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;

wherein the swapped subset of individual parameters of the responder that causes a deviation of the predicted target characteristic in direction of the experimentally measured target characteristic of the non-responder is a biomarker of a responder; wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

Preferably, the deviation is significant or statistically significant.

Also disclosed herein is a computer-implemented method for identifying a biomarker comprising the following steps:
a) Providing patients suffering from the same disease;
b) Obtaining predefined common parameters of each patient, predefined individual parameters of each patient and experimentally measured target characteristic of each patient
c) Classifying the patients into responder and non-responder;
d) Applying a mathematical model on the predefined common parameters and predefined individual parameters of the responder and/or on the predefined common parameters and predefined individual parameters of the non-responder in order to calculate free common parameters and free individual parameters of the responder and free common parameters and free individual parameters of the non-responder;
e) Predicting target characteristic of the responder and predicting target characteristic of the non-responder;
f) Swapping a subset of predefined individual parameters and free individual parameters of the responder between the responder and the non-responder by keeping the free common parameters and predefined common parameters of the responder fixed and/or swapping a subset of predefined individual parameters and free individual parameters of the non-responder between the non-responder and the responder by keeping the free common parameters and predefined common parameters of the non-responder fixed;
g) Predicting target characteristic for the swapped subset of predefined individual parameters and free individual parameters of the responder and/or predicting target characteristic for the swapped subset of predefined individual parameters and free individual parameters of the non-responder;
h) Determining the deviation between the experimentally measured target characteristic of the responder and the predicted target characteristic of the responder for the swapped subset of predefined individual parameters and free individual parameters of the responder and/or determining the deviation between the experimentally measured target characteristic of the non-responder and the predicted target characteristic of the non-responder for the swapped subset of predefined individual parameters and free individual parameters of the non-responder;
wherein a deviation between the experimentally measured target characteristic of the responder and the predicted target characteristic of the responder towards the experimentally measured target characteristic of the non-responder identifies a biomarker of a responder and/or wherein a deviation between the experimentally measured target characteristic of the non-responder and the predicted target characteristic of the non-responder towards the experimentally measured target characteristic of the responder identifies a biomarker of a non-responder.

Alternatively step a) could read as follows:
a) Providing a group of patients having the same disease and receiving equal treatment.

Alternatively in the afore-mentioned method step d) can be drafted as follows:
d) Applying a mechanistic model on the predefined common parameters and predefined individual parameters of the responder and/or on the predefined common parameters and predefined individual parameters of the non-responder in order to calculate free common parameters and free individual parameters of the responder and free common parameters and free individual parameters of the non-responder by minimizing the difference between the experimentally measured target characteristic of the responder and the predicted target characteristic of the responder or between the experimentally measured target characteristic of the non-responder and the predicted target characteristic of the non-responder;

Further disclosed herein is a method for identifying a biomarker comprising the following steps:
a) Providing patients suffering from the same disease;
b) Obtaining predefined common parameters of each patient, predefined individual parameters of each patient and experimentally measured target characteristic of each patient;
c) Classifying the patients into responder and non-responder;
d1) Applying a mathematical model on the predefined common parameters and predefined individual parameters of the responder in order to calculate free common parameters and free individual parameters of the responder by minimizing the difference between the experimentally measured target characteristic of the responder and the predicted target characteristic of the responder;
d2) Applying a mathematical model on the predefined common parameters and predefined individual parameters of the non-responder in order to calculate free common parameters and free individual parameters of the non-responder by minimizing the difference between the experimentally measured target characteristic of the non-responder and the predicted target characteristic of the non-responder;
e1) Predicting target characteristic of the responder;
e2) Predicting target characteristic of the non-responder;
f1) Swapping a subset of predefined individual parameters and free individual parameters of the responder between the responder and the non-responder by keeping the free common parameters and predefined common parameters of the responder fixed;
f2) Swapping a subset of predefined individual parameters and free individual parameters of the non-responder between the non-responder and the responder by keeping the free common parameters and predefined common parameters of the non-responder fixed;
g1) Predicting target characteristic for the swapped subset of predefined individual parameters and free individual parameters of the responder;
g2) Predicting target characteristic for the swapped subset of predefined individual parameters and free individual parameters of the non-responder;
h1) Determining the deviation between the experimentally measured target characteristic of the responder and the predicted target characteristic of the responder for the swapped subset of predefined individual parameters and free individual parameters of the responder;
h2) Determining the deviation between the experimentally measured target characteristic of the non-responder and the predicted target characteristic of the non-responder for the subset of predefined individual parameters and free individual parameters parameter of the non-responder;
i1) wherein a deviation between the experimentally measured target characteristic of the responder and the predicted target characteristic of the responder towards the experimentally measured target characteristic of the non-responder identifies a biomarker of a responder; and/or
i2) wherein a deviation between the experimentally measured target characteristic of the non-responder and the predicted target characteristic of the non-responder towards the experimentally measured target characteristic of the responder identifies a biomarker of a non-responder.

Further disclosed herein is a method for identifying a biomarker comprising the following steps:
a) Providing a group of patients;
b) Obtaining predefined common parameters of each patient, predefined individual parameters of each patient and experimentally measured target characteristic of each patient of the group of patients;
c) Providing a mathematical model;
d) Determining free common parameters and free individual parameters of the mathematical model by entering the predefined common parameters of each patient, the predefined individual parameters of each patient and the experimentally measured target characteristics of each patient of the group of patients into the mathematical model and minimizing the difference between the experimentally measured target characteristics for each patient and the predicted target characteristics for each patient;
e) Selecting a responder and a non-responder from the group of patients;
f) Iteratively swapping one / a / each subset of predefined individual parameters and free individual parameters of the responder between the responder and the non-responder and/or swapping one / a / each subset of predefined individual parameters and free individual parameters of the non-responder between the non-responder and the responder;
g) Predicting target characteristic for the swapped subset of predefined individual parameters and free individual parameters of the responder and/or predicting target characteristic for the swapped subset of predefined individual parameters and free individual parameters of the non-responder;
h) Determining the deviation between the experimentally measured target characteristic of the responder and the predicted target characteristic of the responder for the swapped subset of predefined individual parameters and free individual parameters of the responder and/or determining the deviation between the experimentally measured target characteristic of the non-responder and the predicted target characteristic of the non-responder for the swapped subset of predefined individual parameters and free individual parameters of the non-responder;
wherein a deviation between the experimentally measured target characteristic of the responder and the predicted target characteristic of the responder towards the experimentally measured target characteristic of the non-responder identifies a biomarker of a responder and/or wherein a deviation between the experimentally measured target characteristic of the non-responder and the predicted target characteristic of the non-responder towards the experimentally measured target characteristic of the responder identifies a biomarker of a non-responder.

Also disclosed herein is a method for identifying a biomarker comprises the following steps:
a) Providing a group of patients;
b) Obtaining predefined common parameters of each patient, predefined individual parameters of each patient and experimentally measured target characteristic of each patient of the group of patients;
c) Providing a mathematical model;
d) Determining common parameters and free individual parameters of the mathematical model by entering the predefined common parameters of each patient, the predefined individual parameters of each patient and the experimentally measured target characteristics of each patient of the group of patients into the mathematical model and minimizing the difference between the experimentally measured target characteristic for each patient and the predicted target characteristic for each patient;
e) Selecting a responder and a non-responder from the group of patients;
f1) Swapping one / a / each subset of predefined individual parameters and free individual parameters of the responder between the responder and the non-responder;
f2) Swapping one / a / each subset of predefined individual parameters and free individual parameters of the non-responder between the non-responder and the responder;
g1) Predicting target characteristic for the swapped subset of predefined individual parameters and free individual parameters of the responder;
g2) Predicting target characteristic for the swapped subset of predefined individual parameters and free individual parameters of the non-responder;
h1) Determining the deviation between the experimentally measured target characteristic of the responder and the predicted target characteristic of the responder for the swapped subset of predefined individual parameters and free individual parameters of the responder;
h2) Determining the deviation between the experimentally measured target characteristic of the non-responder and the predicted target characteristic of the non-responder for the swapped subset of predefined individual parameters and free individual parameters of the non-responder;
i1) wherein a deviation between the experimentally measured target characteristic of the responder and the predicted target characteristic of the responder towards the experimentally measured target characteristic of the non-responder identifies a biomarker of a responder, and/or
i2) wherein a deviation between the experimentally measured target characteristic of the non-responder and the predicted target characteristic of the non-responder towards the experimentally measured target characteristic of the responder identifies a biomarker of a non-responder.

Also disclosed herein is a method for identifying a biomarker comprises the following steps:
a) Providing a group of patients;
b) Obtaining predefined common parameters of each patient, predefined individual parameters of each patient and experimentally measured target characteristic of each patient of the group of patients;
c) Selecting a responder and a non-responder from the group of patients;
d) Providing a mathematical model;
e1) Applying the mathematical model on the predefined common parameters and predefined individual parameters of the responder in order to calculate free common parameters and free individual parameters of the responder by minimizing the difference between the experimentally measured target characteristic of the responder and the predicted target characteristic of the responder;
e2) Applying the mathematical model on the predefined common parameters and predefined individual parameters of the non-responder in order to calculate free common parameters and free individual parameters of the non-responder by minimizing the difference between the experimentally measured target characteristic of the non-responder and the predicted target characteristic of the non-responder;
f1) Swapping one / a / each subset of predefined individual parameters and free individual parameters of the responder between the responder and the non-responder;
f2) Swapping one / a / each subset of predefined individual parameters and free individual parameters of the non-responder between the non-responder and the responder;
g1) Predicting target characteristic for the swapped subset of predefined individual parameters and free individual parameters of the responder;
g2) Predicting target characteristic for the swapped subset of predefined individual parameters and free individual parameters of the non-responder;
h1) Determining the deviation between the experimentally measured target characteristic of the responder and the predicted target characteristic of the responder for the swapped subset of predefined individual parameters and free individual parameters of the responder;
h2) Determining the deviation between the experimentally measured target characteristic of the non-responder and the predicted target characteristic of the non-responder for the swapped subset of predefined individual parameters and free individual parameters of the non-responder;
i1) wherein a deviation between the experimentally measured target characteristic of the responder and the predicted target characteristic of the responder towards the experimentally measured target characteristic of the non-responder identifies a biomarker of a responder, and/or
i2) wherein a deviation between the experimentally measured target characteristic of the non-responder and the predicted target characteristic of the non-responder towards the experimentally measured target characteristic of the responder identifies a biomarker of a non-responder.

Further disclosed herein is a method for identifying a biomarker comprises the following steps:
a) Providing a group of patients having the same disease and receiving equal treatment;
b) Obtaining predefined common parameters of each patient, predefined individual parameters of each patient and experimentally measured target characteristic of each patient of the group of patients;
c) Applying a mathematical model on the predefined common parameters of each patient, on the predefined individual parameters of each patient and on the experimentally measured target characteristic of each patient of the group of patients in order to calculate free common parameters and free individual parameters of the mathematical model by minimizing the differences between the experimentally measured target characteristic of each patient and the predicted target characteristic of each patient of the group of patients;
d) Selecting a responder and a non-responder from the group of patients;
e) Swapping one / a / each subset of predefined individual parameters and free individual parameters of the responder between the responder and the non-responder and/or swapping one / a / each subset of predefined individual parameters and free individual parameters of the non-responder between the non-responder and the responder;
f) Predicting target characteristic for the swapped subset of predefined individual parameters and free individual parameters of the responder and/or predicting target characteristic for the swapped subset of predefined individual parameters and free individual parameters of the non-responder;
g) Determining the deviation between the experimentally measured target characteristic of the responder and the predicted target characteristic of the responder for the swapped subset of predefined individual parameters and free individual parameters of the responder and/or determining the deviation between the experimentally measured target characteristic of the non-responder and the predicted target characteristic of the non-responder for the swapped subset of predefined individual parameters and free individual parameters of the non-responder;
wherein a deviation between the experimentally measured target characteristic of the responder and the predicted target characteristic of the responder towards the experimentally measured target characteristic of the non-responder identifies a biomarker of a responder and/or wherein a deviation between the experimentally measured target characteristic of the non-responder and the predicted target characteristic of the non-responder towards the experimentally measured target characteristic of the responder identifies a biomarker of a non-responder.

In a further embodiment the responder is a patient that differs in the experimentally measured target characteristic from the best responding patient by no more than 5% of the difference between the weakest responding patient and the strongest responding patient and the non-responder is a patient that differs in the experimentally measured target characteristic from the weakest responding patient by no more than 5% of the difference between the weakest responding patient and the best responding patient within a group of patients having the same disease and receiving equal treatment.

In another embodiment the responder is a patient that differs in experimentally measured target characteristic from the best responding patient by no more than 10% of the difference between the weakest responding patient and the strongest responding patient and the non-responder is a patient that differs in the experimentally measured target characteristic from the weakest responding patient by no more than 10% of the difference between the weakest responding patient and the best responding patient within a group of patients having the same disease and receiving equal treatment.

The method of identifying biomarkers that indicate a patient's response to a specific treatment according to the invention may be applied to patients of a group of patients having the same disease and receiving equal treatment, wherein the disease belongs to one of the following disease types: diabetes, secondary complications of diabetes, cardiovascular diseases, neurodegenerative diseases infectious diseases, including opportunistic diseases, prion diseases, immunological diseases, autoimmune diseases, cell proliferative diseases, inflammation and stroke.

In one embodiment the inventive method of identifying biomarkers is applied to a group of patients having an infectious disease, including opportunistic disease that is caused by retroviruses, human endogenous retroviruses, lentiviruses, oncoretroviruses, hepadnaviruses, herpesviruses, flaviviridae, and/or adenoviruses.

In another embodiment the inventive method of identifying biomarkers is applied to a group of patients having an infectious disease, including opportunistic disease, which is selected from the group comprising AIDS, Alveolar Hydatid Disease (AHD, Echinococcosis), Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Boreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, Chronic Fatigue Syndrome, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cutaneous Larva Migrans (CLM), Cyclosporiasis (Cyclospora Infection), Cysticercosis (Neurocysticercosis), Cytomegalovirus Infection, Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entomoeba coli Infection, Entomoeba dispar Infection, Entomoeba hartmanni Infection, Entomoeba histolytica Infection (Amebiasis), Entomoeba polecki Infection, Enterobiasis (Pinworm Infection), Enterovirus Infection (Non-Polio), Epstein-Barr Virus Infection, Escherichia coli Infection, Foodborne Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis (HCV, HBV), Herpes Zoster (Shingles), HIV Infection, Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, River Blindness (Onchocerciasis), Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection, Viral Hemorrhagic Fever, Warts, Waterborne infectious Diseases, West Nile Virus Infection (West Nile Encephalitis), Whooping Cough, Yellow Fever, tuberculosis, leprosy, mycobacteria-induced meningitis.

In another embodiment the inventive method of identifying biomarkers is applied to a group of patients having an immunological disease and/or autoimmune disease which is selected from the group comprising: asthma, diabetes, rheumatic diseases, AIDS, rejection of transplanted organs and tissues, rhinitis, chronic obstructive pulmonary diseases, osteoporisis, ulcerative colitis, sinusitis, lupus erythematosus, recurrent infections, atopic dermatitis / eczema and occupational allergies, food allergies, drug allergies, severe anaphylactic reactions, anaphylaxis, manifestations of allergic diseases, primary immunodeficiencies, antibody deficiency states, cell mediated immunodeficiencies, severe combined immunodeficiency, DiGeorge syndrome, Hyper-IgE syndrome, Wiskott-Aldrich syndrome, ataxia-telangiectasia, immune mediated cancers, white cell defects, autoimmune diseases, systemic lupus erythematosus, rheumatoid arthritis (RA), multiple sclerosis (MS), immune-mediated or Type 1 Diabetes Mellitus, immune mediated glomerulonephritis, scleroderma, pernicious anemia, alopecia, pemphigus, pemphigus vulgaris, myasthenia gravis, inflammatory bowel diseases, Crohn's disease, psoriasis, autoimmune thyroid diseases, Hashimoto's disease, dermatomyositis, goodpastture syndrome, myasthenia gravis pseudoparalytica, ophtalmia sympatica, phakogene uveitis, chronical aggressive hepatitis, primary billiary cirrhosis, autoimunehemolytic anemia, Werlhof disease.

In another embodiment of the present invention the inventive method of identifying biomarkers is applied to a group of patients having a cardiovascular disease that is selected from the group consisting of: adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

In a further embodiment of the present invention the inventive method of identifying biomarkers is applied to a group of patients having a proliferative disease that is selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

In a further embodiment of the present invention the inventive method of identifying biomarkers is applied to a group of patients having an inflammatory disease that is selected from the group comprising: abscessation, acanthameba, acanthamebiasis, acne vulgaris, actinomycosis, acute inflammatory dermatoses, acute laryngeal infections of adults, acute multifocal placoid pigmentary epitheliopathy, acute (thermal) injury, acute retinal necrosis, acute suppurative otitis media, algal disorders, allergic contact dermatitis, amyloidosis angioedema, ankylosing spondylitis, aspergillosis, atopic dermatitis, Aujeszky's disease, autoantibodies in vasculitis, babesiosis, bacterial disorders, bacterial laryngitis, bacterial meningitis, Behcet's disease, birdshot choroidopathy, blastomycosis, borna disease, brucellosis, bullous myringitis, bursitis, candidiasis, canine distemper encephalomyelitis, canine distemper encephalomyelitis in immature animals, canine ehrlichiosis, canine herpes virus encephalomyelitis, cholesteatoma, chronic (granulomatous) diseases, chronic inflammatory dermatoses, chronic relapsing encephalomyelitis, chronic suppurative otitis media, cicatricial pemphigoid, coccidiomycosis, coccidioidomycosis, common upper respiratory infection, contact ulcer and granuloma, Crohn's disease, cryptococcosis, cysticercosis, dermatomyositis, diphtheria, discoid lupus erythematosus, drug-induced vasculitis, drug or hypersensitivity reaction, encephalitozoonosis, eosinophilic meningoencephalitis, erythemal multiforme (EM minor), feline leukemia virus, feline immunodeficiency virus, feline infectious peritonitis, feline polioencephalomyelitis, feline spongiform encephalopathy, fibromyositis, Fuch's heterochromic cyclitis, gastroesophageal (laryngopharyngeal) reflux disease, giant cell arteritis, glanders, glaucomatocyclitic crisis, gonorrhea granular myringitis, granulomatous meningoencephalomyelitis, herpes simplex, histoplasmosis, idiopathic diseases, idiopathic inflammatory disorders, immune and idiopathic disorders, infections of the immunocompromised host, infectious canine hepatitis, inhalation laryngitis, interstitial nephritis, irritant contact dermatitis, juvenile rheumatoid arthritis, Kawasaki's disease, La Crosse virus encephalitis, laryngeal abscess, laryngotracheitis (croup), leishmaniasis, lens-induced uveitis, leprosy, leptospirosis, leukemia, lichen planus, lupus, lyme disease, lymphoma, meningitis, meningoencephalitis in greyhounds, miscellaneous meningitis / meningoencephalitis, microscopic polyangiitis, multifocal choroiditis, multifocal distemper encephalomyelitis in mature animals, multiple sclerosis, muscle tension dysphonias, mycotic (fungal) diseases, mycotic diseases of the CNS, necrotizing encephalitis, neosporosis, old dog encephalitis, onchocerciasis, parasitic encephalomyelitis, parasitic infections, pars planitis, parvovirus encephalitis, pediatric laryngitis, pollution and inhalant allergy, polymyositis, post-vaccinal canine distemper encephalitis, post-vaccinal rabies, prion protein induced diseases, protothecosis, protozoal encephalitis-encephalomyelitis, psoriasis, psoriatic arthritis, pug dog encephalitis, pyogranulomatous meningoencephalomyelitis, rabies, radiation injury, radiation laryngitis, radionecrosis, relapsing polychondritis, Reiters's syndrome, retinitis pigmentosa, retinoblastoma, rheumatoid arthritis, rickettsial disorders, rocky mountain spotted fever, salmon poisoning, sarcocystosis, sarcoidosis, schistosomiasis, scleroderma, scleroma, serpiginous choroiditis, shaker dog disease, Sjogren's syndrome, spasmodic croup, spirochetal (syphilis) diseases, spongiotic dermatitis, sporotrichosis, steroid responsive meningitis-arteritis, Stevens-Johnson syndrome (SJS, EM major), supraglottitis (epiglottitis), sympathetic ophthalmia, syngamus laryngeus, syphilis, systemic lupus erythematosus, systemic vasculitis in sarcoidosis, Takayasu's arteritis, tendinitis (tendonitis), thromboangiitis obliterans (Buerger's Disease), tick-borne encephalitis in dogs, toxic epidermal necrolysis (TEN), toxocariasis, toxoplasmosis, trauma, traumatic laryngitis, trichinosis, trypanosomiasis, tuberculosis, tularemia, ulcerative colitis, urticaria (hives), vasculitis, vasculitis and malignancy, vasculitis and rheumatoid arthritis, vasculitis in systemic lupus erythematosus, vasculitis in the idiopathic inflammatory myopathies, vasculitis of the central nervous system, vasculitis secondary to bacterial, fungal, and parasitic infection, viral disorders, viral laryngitis, vitiligo, vocal abuse, vocal-cord hemorrhage, Vogt Koyanagi Harada syndrome, Wegener's granulomatosis, and Whipple's disease.

In another embodiment of the present invention the inventive method of identifying biomarkers is applied to a group of patients having a neurodegenerative disease that is selected from the group comprising: Alzheimer disease, Parkinson disease, Huntington disease, amyotrophic lateral sclerosis, AIDS-related dementia, retinitis pigmentosa, spinal muscular atrophy and cerebrellar degeneration, fragile X-associated tremor/ataxia syndrome (FXTAS), progressive supranuclear palsy (PSP), and striatonigral degeneration (SND), which is included with olivopontocerebellear degeneration (OPCD), and Shy Drager syndrome (SDS) in a syndrome known as multiple system atrophy (MSA).

In another embodiment of the present invention the inventive method of identifying biomarkers is applied to a group of patients having a neurodegenerative disease and receiving a treatment with a pharmaceutical composition that comprise at least one drug selected from the group comprising meclofenoxate, nicergoline, piracetame, pyritinole, tacrine, donezepil, galantamine, rivastigmine, memantine, IMPase-inhibitors and extracts from Gingko biloba.

In a further embodiment of the present invention the inventive method of identifying biomarkers is applied to a group of patients having a proliferative disease and receiving a treatment with an antiproliferative agent that is selected from the group comprising sorafinib (Nexavar^{®}), chlorethamine, cyclophosphamide (cytoxan), trofosfamide, ifosfamide (Ifex), melphalan (Alkeran), mechlorethamine hydrochloride (Mustargen), chlorambucil (Leukeran), busulfan (Myleran), thiotepa, 2-cyano-3-(3,4-dihydroxyphenyl)-N-(phenylmethyl)-2-propenamide, carmustine, carboplalomustine, dacarbazine, procarbazine, erlotinib, temozolomide, treosulfan, estramustine, N-(3-(5-chloro-2-(2-methoxy-4-(4-methylpiperazin-1-yl)phenylamino)pyrimidin-4-yloxy)phe-nyl)acrylamide, nimustine, methotrexate (MTX, Mexate), 5-fluorouracil (Fluoracil, 5-FU), 6-thioguanine (Thioguanine, 6-TG), 6-mercaptopurine, (N-[-4-[(3-Chloro-4-fluorophenyl)amino]-7-[3-(4-morpholinyl)propoxy]-6-quinazolinyl]-2-propenamide), sunitinib, N-(3-chlorophenyl)-6,7-dimethoxy-4-quinazolinamine, fludarabine (Fludara), floxuridine (FUDR), cladribine, pentostatin, gemcitabine (Gemzar), cytarabine, azathioprine, raltitrexed, capecitabine (Xeloda), 4-phenethylamino-6-(yderoxyl)phenyl-7H-pyrrolo(2,3-d)pyrimidine, cytosine arabinoside, deoxycoformycin (Pentostatin, Nipent), lapatinib, thioguanine, 7-(4-(3-ethynylphenylamino)-7-methoxyquinazolin-6-yloxy)-N-hydroxyheptanamide, mercaptopurine (6-MP, Purinethol), paclitaxel, docetaxel, hydroxycarbamide (hydroxyurea), imatinibe, Miltefosine^{®}, N-(3-(5-chloro-2-(4-(4-methylpiperazin-1-yl)phenylamino)pyrimidin-4-yloxy)phenyl)acryl-amide, cetuximab, amsacrine, 4-[(3-Bromophenyl)amino]-6,7-dimethoxyquinazoline hydrochloride), pentostatin, bexarotene, tretinoin, asparaginase, trastuzumab (also known as Herceptin^{®}), alemtuzumab (also known as MabCampath^{®}), rituximab (also known as MabThera^{®}), N-[4-[(3-Chloro-4-fluorophenyl)amino]-7-[[(3"S")-tetrahydro-3-furanyl]oxy]-6-quinazolinyl]-4(dimethylamino)-2-butenamide, glucocorticoids (prednisone), hydrocortisones, gefitinib, dexamethasones (Decadron), (E)-N-(4-((3-chloro-4-fluorophenyl)amino)-3-cyano-7 -ethoxyquinol in-6-yl)-4-(dimethylamino)but-2-enamide, estrogens [fosfestrol, estramustine (Emcyt), chlorotrianisene (TACE), diethylstilbestrol (DES)], (R)-6-(4-((4-methylpiperazin-1-yl)methyl)phenyl)-N-(1-phenylethyl)-7H-pyrrolo[2,3-d]pyrimidin-4-amine, aromatase inhibitors [anastrozole (Arimidex)], LHRH (buserelin, goserelin, leuprorelin, triptorelin), N-(3-Chloro-4-((3-fluorophenyl)methoxy)phenyl)-6-(5-(((2-(methylsulfonyl)ethyl)amino)methyl)-2-furanyl)-4-quinazolinamine bis(4-methylbenzenesulfonate), flutamide (Eulexing), bicalutamide (Casodex), leuprolide (Lupron), goserelin acetate (Zoladex), [4-[[1-(3-fluorophenyl)methyl]-1H-indazol-5-ylamino]-5-methylpyrrolo[2,1-f][1,2,4]triazin-6-yl]carbamic acid (3S)-3-Morpholinylmethyl ester, vandetanib, cyproterone acetate, N-(3-(5-chloro-2-(4-(4-methylpiperazin-1-yl)phenylamino)pyrimidin-4-ylthio)phenyl)-acrylamide, panitumumab, progestine (Medoxyprogesterone acetate [(Depo-provera)], megestrol acetate (Megacel), tamoxifen and toremifen, daunorubicin (Daunomycin, Cerubidine), doxorubicin (adriamycin), liposomal adriamycin, dactinomycin, mitomycin C, (R,Z)-5-((2,6-dichlorobenzyl)sulfonyl)-3-((3,5-dimethyl-4-(2-(pyrrolidin-1-ylmethyl)pyrrolidine-1-carbonyl)-1H-pyrrol-2-yl)methylene) indolin-2-one, bleomycin (Blenoxane), epirubicin (4-epi-adriamycin), idarubicin (Idamycin), AMG102 (Amgen, Inc), dactinomycin (Actinomycin D, Cosmegen), mitoxantrone (Novantrone), AMG-208 (Amgen, Inc), mitomycin C (Mitomycin), plicamycin (Mithracin), amsacrine, actinomycin D, vincristine, vinblastine, SCH900105 (AV-299) (Aveo Pharmaceuticals Inc.), vindesine, etoposide, XL184 (Exelixis, Inc), N-((2R)-1,4-Dioxan-2-ylmethyl)-N-methyl-N'-[3-(1 -methyl-1H-pyrazol-4-yl)-5-oxo-5H-benzo[4,5]cyclohepta[1,2-b]pyridin-7-yl]sulfamide, PF-04254644 (Pfizer, Inc), N-(1,3-Benzodioxol-5-ylmethyl)-4-benzofuro[3,2-D]pyrimidin-4-yl-1-piperazinecarbothioamide, teniposide, cisplatin, carboplatin, 9S-(9α,10β,12α))-2,3,9,10,11,12-hexahydro-10-hydroxy-10-(methoxycarbonyl)-9 methyl 9,12 epoxy 1H diindolo-[1,2,3 fg:3',2',1' kl]pyrrolo[3,4 i][1,6]benzodiazocin-1-one, oxaliplatin, vinorelbine, etoposide (VP-16, Etopophos, VePesid), ARQ197 (ArQule, Inc), teniposide (VM-26, Vumon) topotecan (Hycamtin), irinotecan (Camptosar), carmustine (BCNU), lomustine (CCNU), JNJ-38877605 (Johnson & Johnson, Inc), streptozocin (Zanosar), 1,3-Dihydro-3-[(3,5-dimethyl-1H-pyrrol-2-yl)methylene]-2H-indol-2-one, camptothecin, topotecan, SGX-523 (SGX Pharmaceuticals, Inc), irinotecan, aminoglutethimide, formestane, (3Z)-N-(3-Chlorophenyl)-3-({3,5-dimethyl-4-[(4-methylpiperazin-1-yl)carbonyl]-1H-pyrrol-2-yl}methylene)-N-methyl-2-oxo-2,3-dihydro-1H-indole-5-sulfonamide, XL880 (Exelixis, Inc), exemestane, letrozole, anastrozole, MGCD-265 (MethylGene, Inc), interleukin-2, interferon-a, genestein, erythropoietin, G-CSF, ibritumomab (Zevalin^{®}), levamisole, asparaginase (Elspar), pegaspargase (Oncaspar), hydroxyurea (Hydrea, Mylocel), 2-(4-(1-(quinolin-6-ylmethyl)-1H-[1,2,3]triazolo[4,5-b]pyrazin-6-yl)-1H-pyrazol-1-yl)ethanol, procarbazine (Matulane) and imatinib mesylate (Gleevec), Neovastat, bevamizumab, avastin, angiozyme, 3-[(1R)-1-(2,6-dichloro-3-fluorophenyl)ethoxy]-5-(1-piperidin-4-ylpyrazol-4-yl)pyridin-2-amine, endostatin, angiostatin, combrestatin, vitaxin, geldanamycin carboxyamido thiazole, celecoxib and tirapazamine.

In another embodiment the inventive method of identifying biomarkers is applied to a group of patients having an infectious disease, including opportunistic disease, receiving a treatment with an anti-infective therapeutic agent that is selected from the following group comprising: Ampicillin, bacampicillin, carbenicillin, indanyl, mezlocillin, piperacillin, ticarcillin, amoxicillin-clavulanic acid, ampicillin-sulbactam, benzylpenicillin, cloxacillin, dicloxacillin, methicillin, oxacillin, penicillin G, penicillin V, piperacillin, tazobactam, ticarcillin, clavulanic acid, nafcillin, cephalosporin of the first generation, cefadroxil, cefazolin, cephalexin, cephalothin, cephapirin, cephradine, cefaclor, cefamandol, cefonicid, cefotetan, cefoxitin, cefprozil, ceftmetazole, cefuroxime, loracarbef, cefdinir, ceftibuten, cefoperazone, cefixime, cefotaxime, cefpodoxime proxetil, ceftazidime, ceftizoxime, ceftriaxone, cefepime, azithromycin, clarithromycin, clindamycin, dirithromycin, erythromycin, roxithromycin, telithromycin, cethromycin, spiramycin, ansymacin, oelandomycin, lincomycin, troleandomycin, cinoxacin, ciprofloxacin, enoxacin, gatifloxacin, grepafloxacin, levofloxacin, lomefloxacin, moxifloxacin, nalidixic, acid, norfloxacin, ofloxacin, sparfloxacin, trovafloxacin, oxolinic, acid, gemifloxacin, perfloxacin, imipenem-cilastatin, meropenem, aztreonam, amikacin, gentamicin, kanamycin, neomycin, netilmicin, streptomycin, tobramycin, paromomycin, teicoplanin, vancomycin, demeclocycline, doxycycline, methacycline, minocycline, oxytetracycline, tetracycline, chlortetracycline, tigecycline, mafenide, silver sulfadiazine, sul-facetamide, sulfadiazine, sulfamethoxazole, sulfasalazine, sulfisoxazole, trimethoprim-sulfamethoxazole, sulfamethizole, rifabutin, rifampin, rifapentine, linezolid, streptogramins, quinopristin, dalfopristin, bacitracin, chloramphenicol, fosfomycin, isoniazid, methenamine, metronidazol, mupirocin, nitrofurantoin, nitrofurazone, novobiocin, polymyxin, spectinomycin, trimethoprim, colistin, cycloserine, capreomycin, ethionamide, pyrazinamide, para-aminosalicyclic acid, erythromycin-2-acetate, erythromycin-2-stearate, erythromycin estolate, erythromycin ethylsuccinate, erythromycin glutamate, erythromycin lactic propionate, rifampicin, miconazole, ketoconazole, clotrimazole, econazole, bifonazole, butoconazole, fenticonazole, isoconazole, oxiconazole, sertaconazole, sulconazole, tioconazole, fluconazole, itroconauzole, isavuconazole, ravuconazole, posaconazole, voriconazole, teraconazole, abfungin, terbinafine, amorolfine, naftifine, butenafine, anidulafungin, caspofungin, micafungin, benzoic acid, ciclopirox olamine, tolnaftate, undecyclenic acid, 5-fluorocytosine, griseofulvin, haloprogin, fusidic acid, gramicidin, pristinamycin, ramoplanin, tyrotricin, nata-mycin, rimocidin, filipin, nystatin, amphotericin B, candicin, hamycin, silver, copper and/or mixtures of the aforementioned agents.

In one embodiment of the present invention the method of identifying a biomarker of a responder and a biomarker of a non-responder with a mechanistic model using individual parameters of the responder, individual parameters of the non-responder, experimentally measured target characteristic of the responder and/or experimentally measured target characteristic of the non-responder comprises the following steps:
I) Iteratively swapping a subset of individual parameters between the responder and the non-responder and predicting target characteristics for each swapped subset of individual parameters of the responder and/or for each swapped subset of individual parameters of the non-responder;
II) Determining deviations between the experimentally measured target characteristic of the responder and the predicted target characteristics for each swapped subset of individual parameters of the responder and/or between the experimentally measured target characteristic of the non-responder and the predicted target characteristics for each swapped subset of individual parameters of the non-responder;

wherein a deviation between the experimentally measured target characteristic of the responder and the predicted target characteristic for the swapped subset of individual parameters of the responder towards the experimentally measured target characteristic of non-responder identifies a biomarker of a responder;
wherein a deviation between the experimentally measured target characteristic of the non-responder and the predicted target characteristic for the swapped subset of individual parameters of the non-responder towards the experimentally measured target characteristic of responder identifies a biomarker of a non-responder;
wherein the responder and the non-responder have a cancer disease and are treated equally against cancer; and
wherein the identified biomarker is a biomarker for the treatment response of a patient having a cancer disease.

### Description of the figures:

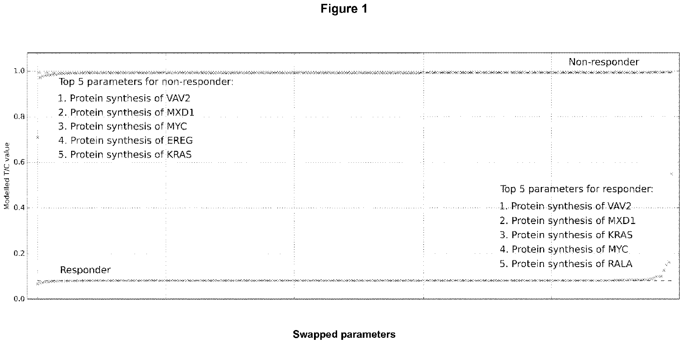

**Figure 1** shows the result of an example of the inventive method for identifying new biomarkers. In this example the method is applied to a responder (state B) and a non-responder (state A) patients having colon cancer and receiving equal treatment with the drug cetuximab. On the X-axis - the swapped individual parameters are represented and on the Y-axis - the T/C values (target characteristic) are drawn. The dashed blue and red lines represent the T/C values of the non-responder and responder, respectively. Blue and red crosses represent the T/C values with swapped individual parameters of the non-responder and responder, respectively. The order of the swapped individual parameters on the X-axis is different for responder and non-responder: both have an ascending order according to the modeled T/C values. The top five biomarkers for the responder and the non-responder are listed.

### EXAMPLES

### Example 1: Identification of new biomarkers for the treatment of colon cancer patients with cetuximab

For this example a mechanistic model of a virtual patient was used. The model is manually curated based on the information from pathway databases and literature. The model is composed of objects representing biological entities such as genes, proteins, protein complexes, phosphorylated proteins, etc. These objects are connected by reactions that represent biochemical reactions or cellular processes, such as the phosphorylation of a protein where the non-phosphorylated protein is a substrate (reactant) of the reaction, the phosphorylated protein is the product (educt) and the reaction is catalyzed by an enzyme (catalyst). Overall the model used in this example comprises 473 genes that are converted into their respective proteins. The synthesis rates of the respective proteins are defined by the predefined parameters of each patient which also include expression data of the individual tumor. Furthermore, the model integrates 345 gain-of-function or loss-of-function mutations for some of these genes that are taken into account if the respective mutation was identified in the individual patient. Proteins subsequently can form protein complexes, undergo protein modifications or decay. The implemented model covers most relevant cancer related signal transduction pathways. Some downstream objects of these pathways act as readouts that are used to estimate a target characteristic. In this example treatment vs. control ratio (T/C value) as measured in xenograft experiments during drug treatment of patient-(tumor-)derived xenograft (PDX) mouse models was used as a target characteristic. A distance between modeled T/C values and experimentally measured T/C values was minimized during optimization of free parameters of the model. A patient was considered as a responder (state B), if the corresponding T/C value was below 0.3. A patient was considered as non-responder (state A), if the corresponding T/C value was above 0.7.

From the mathematical point of view the model used in this example represents a system of ordinary differential equations. The model takes predefined parameters of the untreated patient which include gene expression and mutations as well as drug concentrations as an input and produces an estimate of the target characteristic (T/C value). Response to the single drug cetuximab (a monoclonal antibody that is selectively directed against EGFR) was considered. The model contains thousands of parameters. Although some fraction of these parameters for each patient can be defined from the available experimental data (predefined parameters), most of them cannot be effectively measured experimentally. These free parameters of the model were fixed to the same values for all patients (so in this example all free parameters are free common parameters). The free parameters were estimated using gradient based optimization so that the difference between modeled and experimental T/C values for all available patients is minimized simultaneously. One responder (state B) with experimental T/C value around 0.99 and one non-responder (state A) with experimental T/C value around 0.08 were selected; both were accurately predicted by the model. Values of individual parameters (gene expression and mutation) were swapped between responder and non-responder one by one and corresponding estimations of T/C value were predicted. Other subsets of individual parameters (those which comprise more than one parameter) were not swapped.

**Figure 1** demonstrates the result of this experiment, i.e. experimentally measured T/C values and modeled T/C values. The X-axis represents the individual parameters that were swapped and the Y-axis shows the modeled T/C value. Blue color indicates non-responder and red indicates responder. Dashed lines represent measured T/C values and crosses represent predicted T/C values when the corresponding individual parameter is swapped. The order of the swapped individual parameters on the X-axis is different for responder and non-responder: both have an ascending order according to the predicted T/C values. As an example, the effect of VAV2 expression swapping (which has the strongest effect on the modeled T/C value both for responder and non-responder) is represented by the rightmost red cross and leftmost blue cross. As can be seen the majority of parameters have no significant impact on the modeled T/C value (crosses follow the T/C value of the dashed line), while several of them have notable effect shifting responder towards non-responder or vice versa. Five genes both for responder and non-responder for which swapping of expression results in the strongest change of the predicted T/C value are also listed in **Figure 1**. Therefore these genes can be considered as potential biomarkers of patient to be a responder/non-responder (state B/state A) to cetuximab.

The inventive method identifies EREG and KRAS as among the top-5 candidates with the most significant impact on response predictions, which is in accordance with current knowledge. Furthermore, the method predicts VAV2 as one of the most significant biomarkers for cetuximab response. VAV2 regulates EGFR endocytosis and degradation (Oncogene. 2010; 29(17):2528-2539.) and the VAV2 function has been connected to cetuximab mediated EGFR blockade in the context of breast cancer cell migration/metastasis formation in cells with low and medium expression levels of EGFR (Clin Cancer Res. 2008; 74(79/6161-6170).

## Claims

1. A computer-implemented method of identifying a biomarker of a responder and a biomarker of a non-responder with a mechanistic model using individual parameters of the responder, individual parameters of the non-responder, experimentally measured target characteristic of the responder and experimentally measured target characteristic of the non-responder comprising the following steps:
I) Iteratively swapping a subset of individual parameters between the responder and the non-responder and predicting target characteristics for each swapped subset of individual parameters of the responder and predicting target characteristics for each swapped subset of individual parameters of the non-responder;
II) Determining deviations between the experimentally measured target characteristic of the responder and the predicted target characteristics for each swapped subset of individual parameters of the responder and determining deviations between the experimentally measured target characteristic of the non-responder and the predicted target characteristics for each swapped subset of individual parameters of the non-responder;
wherein the swapped subset of individual parameters of the responder that causes a deviation of the predicted target characteristic in direction of the experimentally measured target characteristic of the non-responder is a biomarker of a responder;
wherein the swapped subset of individual parameters of the non-responder that causes a deviation of the predicted target characteristic in direction of the experimentally measured target characteristic of the responder is a biomarker of a non-responder;
wherein the responder and the non-responder are patients suffering from the same disease and receiving equal treatment; and
wherein the responder exhibits in comparison to the non-responder a difference in the experimentally measured target characteristic which indicates that the treatment of the disease is successful and wherein the non-responder exhibits in comparison to the responder a difference in the experimentally measured target characteristic which indicates that the treatment of the disease is not successful.

2. The method according to claim 1, wherein the deviation is a statistically significant deviation.

3. The method according to claim 1 or 2, wherein the responder and non-responder are patients suffering from proliferative disease.

4. The method according claim 3, wherein the proliferative disease is selected from the group comprising or consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

5. The method according to any one of claims 1 - 4, wherein the individual parameters of the responder and the individual parameters of the non-responder are selected from gene expression data, mutation data, gene allele and abundance of proteins.

6. The method according to any one of claims 1 - 5, wherein the mechanistic model is capable of predicting the target characteristic of the responder from the individual parameters of the responder and wherein the mechanistic model is capable of predicting the target characteristic of the non-responder from the individual parameters of the non-responder.

7. The method according to any one of claims 1 - 6, wherein the mechanistic model consists of a set of ordinary differential equations.

## Patentansprüche

1. Ein computerimplementiertes Verfahren zur Identifizierung eines Biomarkers eines Responders und eines Biomarkers eines Non-Responders mit einem mechanistischen Modell unter Verwendung individueller Parameter des Responders, individueller Parameter des Non-Responders, experimentell gemessener Zielmerkmale des Responders und experimentell gemessener Zielmerkmale des Non-Responders, umfassend die folgenden Schritte:
I) Iteratives Austauschen einer Teilmenge von individuellen Parametern zwischen dem Responder und dem Non-Responder und Vorhersagen von Zielmerkmalen für jede ausgetauschte Teilmenge von individuellen Parametern des Responders und Vorhersagen von Zielmerkmalen für jede ausgetauschte Teilmenge von individuellen Parametern des Non-Responders;
II) Bestimmen von Abweichungen zwischen dem experimentell gemessenen Zielmerkmal des Responders und den vorhergesagten Zielmerkmalen für jede vertauschte Teilmenge von individuellen Parametern des Responders und Bestimmen von Abweichungen zwischen dem experimentell gemessenen Zielmerkmal des Non-Responders und den vorhergesagten Zielmerkmalen für jede vertauschte Teilmenge von individuellen Parametern des Non-Responders;
wobei die vertauschte Teilmenge von individuellen Parametern des Responders, die eine Abweichung des vorhergesagten Zielmerkmals in Richtung des experimentell gemessenen Zielmerkmals des Non-Responders verursacht, ein Biomarker eines Responders ist;
wobei die ausgetauschte Teilmenge von individuellen Parametern des Non-Responders, die eine Abweichung des vorhergesagten Zielmerkmals in Richtung des experimentell gemessenen Zielmerkmals des Responders verursacht, ein Biomarker eines Non-Responders ist;
wobei der Responder und der Non-Responder Patienten sind, die an der gleichen Krankheit leiden und die gleiche Behandlung erhalten; und
wobei der Responder im Vergleich zu dem Non-Responder einen Unterschied in dem experimentell gemessenen Zielmerkmal aufweist, der anzeigt, dass die Behandlung der Krankheit erfolgreich ist, und wobei der Non-Responder im Vergleich zu dem Responder einen Unterschied in dem experimentell gemessenen Zielmerkmal aufweist, der anzeigt, dass die Behandlung der Krankheit nicht erfolgreich ist.

2. Das Verfahren gemäß Anspruch 1, wobei die Abweichung eine statistisch signifikante Abweichung ist.

3. Das Verfahren gemäß Anspruch 1 oder 2, wobei der Responder und der Non-Responder Patienten sind, die an einer proliferativen Erkrankung leiden.

4. Das Verfahren gemäß Anspruch 3, wobei die proliferative Erkrankung aus der Gruppe umfassend oder bestehend aus ausgewählt wird: Adenokarzinom, choroidales Melanom, akute Leukämie, Akustikusneurinom, Ampullarkarzinom, Analkarzinom, Astrozytom, Basalzellkarzinom, Bauchspeicheldrüsenkrebs, Desmoidtumor, Blasenkrebs, Bronchialkarzinom, Brustkrebs, Burkitt-Lymphom, Korpuskarzinom, CUP-Syndrom (Karzinom unbekannter Herkunft), Darmkrebs, Dünndarmkrebs, Dünndarmtumoren, Eierstockkrebs, Endometriumkarzinom, Ependymom, Epithelkrebsarten, Ewing-Tumoren, Magen-Darm-Tumoren, Magenkrebs, Gallenblasenkrebs, Gallenblasenkarzinome, Gebärmutterkrebs, Gebärmutterhalskrebs, Glioblastome, gynäkologische Tumore, HNO-Tumore, hämatologische Neoplasien, Haarzellenleukämie, Harnröhrenkrebs, Hautkrebs, Hodenkrebs, Hirntumore (Gliome), Hirnmetastasen, Hodenkrebs, Hypophysentumor, Karzinoide, Kaposi-Sarkom, Kehlkopfkrebs, Keimzelltumor, Knochenkrebs, kolorektales Karzinom, Kopf-Hals-Tumoren (Tumoren im Hals-Nasen-Ohren-Bereich), Kolonkarzinom, Kraniopharyngeom, Mundhöhlenkrebs (Krebs im Mundbereich und an den Lippen), Krebs des Zentralnervensystems, Leberkrebs, Lebermetastasen, Leukämie, Lidtumor, Lungenkrebs, Lymphdrüsenkrebs (Hodgkin/Non-Hodgkin), Lymphome, Magenkrebs, malignes Melanom, bösartige Neoplasie, bösartige Tumoren des Magen-Darm-Trakts, Brustkrebs, Rektumkarzinom, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, Osteolytische Karzinome und osteoplastische Karzinome, Osteosarkome, Ovarialkarzinom, Pankreaskarzinom, Peniskrebs, Plasmozytom, Prostatakrebs, Rachenkrebs, Rektumkarzinom, Retinoblastom, Vaginalkrebs, Schilddrüsenkarzinom, Schneeberger-Krankheit, Speiseröhrenkrebs, Spinaliome, T-Zell-Lymphom (Mycosis fungoides), Thymom, Tubenkarzinom, Augentumore, Harnröhrenkrebs, urologische Tumore, Urothelkarzinom, Vulvakrebs, Warzenbildung, Weichteiltumore, Weichteilsarkom, Wilm-Tumor, Gebärmutterhalskrebs und Zungenkrebs.

5. Das Verfahren gemäß einem der Ansprüche 1 - 4, wobei die individuellen Parameter des Responders und die individuellen Parameter des Non-Responders aus Genexpressionsdaten, Mutationsdaten, Genallelen und Proteinhäufigkeiten ausgewählt werden.

6. Das Verfahren gemäß einem der Ansprüche 1 - 5, wobei das mechanistische Modell in der Lage ist, das Zielmerkmal des Responders aus den individuellen Parametern des Responders vorherzusagen und wobei das mechanistische Modell in der Lage ist, das Zielmerkmal des Non-Responders aus den individuellen Parametern des Non-Responders vorherzusagen.

7. Das Verfahren gemäß einem der Ansprüche 1 - 6, wobei das mechanistische Modell aus einer Reihe von gewöhnlichen Differentialgleichungen besteht.

## Revendications

1. Une méthode informatisée d'identification d'un biomarqueur de réponse et d'un biomarqueur de non-réponse avec un modèle mécaniste utilisant des paramètres individuels du patient répondeur, des paramètres individuels du patient non répondeur, une caractéristique cible mesurée expérimentalement du patient répondeur et une caractéristique cible mesurée expérimentalement du patient non répondeur, comprenant les étapes suivantes :
I) permutation itérative d'un sous-ensemble de paramètres individuels entre le patient répondeur et le patient non répondeur, et prédiction des caractéristiques cibles pour chaque sous-ensemble permuté de paramètres individuels du patient répondeur et pour chaque sous-ensemble permuté de paramètres individuels du patient non répondeur ;
II) détermination des écarts entre la caractéristique cible mesurée expérimentalement du patient répondeur et les caractéristiques cibles prédites pour chaque sous-ensemble permuté de paramètres individuels du patient répondeur, et des écarts entre la caractéristique cible mesurée expérimentalement du patient non répondeur et les caractéristiques cibles prédites pour chaque sous-ensemble permuté de paramètres individuels du patient non répondeur ;
où le sous-ensemble permuté de paramètres individuels du patient répondeur qui induit un écart de la caractéristique cible prédite se rapprochant de la caractéristique cible mesurée expérimentalement du patient non répondeur constitue un biomarqueur de réponse ;
où le sous-ensemble permuté de paramètres individuels du patient non répondeur qui induit un écart de la caractéristique cible prédite se rapprochant de la caractéristique cible mesurée expérimentalement du patient répondeur constitue un biomarqueur de non-réponse ;
où le patient répondeur et le patient non répondeur sont des patients souffrant de la même maladie et recevant le même traitement ; et
où le patient répondeur présente, par rapport au patient non répondeur, une différence dans la caractéristique cible mesurée expérimentalement qui indique que le traitement de la maladie est efficace, et où le patient non répondeur présente, par rapport au patient répondeur, une différence dans la caractéristique cible mesurée expérimentalement qui indique que le traitement de la maladie n'est pas efficace.

2. La méthode conforme à la revendication 1, où l'écart est un écart statistiquement significatif.

3. La méthode conforme à la revendication 1 ou 2, où le patient répondeur et le patient non répondeur sont des patients souffrant d'une maladie proliférative.

4. La méthode conforme à la revendication 3, où la maladie proliférative est sélectionnée dans le groupe incluant ou constitué des maladies suivantes : adénocarcinome, mélanome choroïdien, leucémie aiguë, neurinome de l'acoustique, carcinome ampullaire, carcinome anal, astrocytome, carcinome basocellulaire, cancer du pancréas, tumeur desmoïde, cancer de la vessie, carcinome bronchique, cancer du sein, lymphome de Burkitt, cancer du corps utérin, syndrome de CAPI (cancer de primitif inconnu), cancer colorectal, cancer de l'intestin grêle, tumeurs de l'intestin grêle, cancer des ovaires, carcinome de l'endomètre, épendymome, cancers épithéliaux, tumeurs d'Ewing, tumeurs gastro-intestinales, cancer de l'estomac, cancer de la vésicule biliaire, carcinomes de la vésicule biliaire, cancer de l'utérus, cancer du col de l'utérus, glioblastomes, tumeurs gynécologiques, tumeurs ORL, néoplasies hématologiques, leucémie à trycholeucocytes, cancer de l'urètre, cancer de la peau, cancer du scrotum, tumeurs cérébrales (gliomes), métastases cérébrales, cancer des testicules, tumeur de l'hypophyse, carcinoïdes, sarcome de Kaposi, cancer du larynx, tumeur germinale, cancer des os, carcinome colorectal, tumeurs de la tête et du cou (tumeurs de la sphère ORL), carcinome du côlon, craniopharyngiomes, cancer de la cavité buccale (cancer dans la zone de la bouche et des lèvres), cancer du système nerveux central, cancer du foie, métastases hépatiques, leucémie, tumeur de la paupière, cancer du poumon, cancer des ganglions, lymphomes (hodgkiniens/non hodgkiniens), cancer de l'estomac, mélanome malin, néoplasie maligne, tumeurs malignes du tractus gastro-intestinal, carcinome du sein, cancer du rectum, médulloblastomes, mélanome, méningiomes, maladie de Hodgkin, mycosis fongoïde, cancer du nez, neurinome, neuroblastome, cancer du rein, hypernéphrome, lymphomes non hodgkiniens, oligodendrogliome, carcinome de l'œsophage, carcinomes ostéolytiques et ostéoplastiques, ostéosarcomes, carcinome ovarien, carcinome pancréatique, cancer du pénis, plasmocytome, cancer de la prostate, cancer du pharynx, carcinome du rectum, rétinoblastome, cancer du vagin, carcinome thyroïdien, maladie de Schneeberg, cancer de l'œsophage, spinaliomes, lymphome à cellules T (mycosis fongoïde), thymome, carcinome des trompes, tumeurs oculaires, cancer de l'urètre, tumeurs urologiques, carcinome urothélial, cancer de la vulve, aspect verruqueux, tumeurs des tissus mous, sarcome des tissus mous, tumeur de Wilm, carcinome du col de l'utérus et cancer de la langue.

5. La méthode conforme à l'une des revendications 1 à 4, où les paramètres individuels du patient répondeur et les paramètres individuels du patient non répondeur sont choisis parmi les données d'expression génétique, de mutation, d'allèles et d'abondance des protéines.

6. La méthode conforme à l'une des revendications 1 à 5, où le modèle mécaniste a la capacité de prédire la caractéristique cible du patient répondeur à partir de ses paramètres individuels et la caractéristique cible du patient non-répondeur à partir de ses paramètres individuels.

7. La méthode conforme à l'une des revendications 1 à 6, où le modèle mécaniste consiste en un ensemble d'équations différentielles ordinaires.
